# DEMANDE DE BREVET EUROPEEN

(11) **EP 1 443 044 A1**
(43) Date de publication de la demande: **04.08.2004**
(21) Numéro de dépôt: 03356014.5
(22) Date de dépôt: 03.02.2003
(51) Int. Cl.: C07D 311/54

(54) **Procédés de préparation de dérivés de iodo-chromones fongicides**

(71) Demandeur: Bayer CropScience SA, 69266 Lyon Cedex 09 (FR)
(72) Inventeur: Delamare, Madelaine, 69003 Lyon (FR); Casado, Michel, 69360 ST Symphorien d Ozon (FR)
(74) Mandataire: Mérigeault, Shona

(57) **Abrégé**

Cette invention concerne de nouveaux procédés de préparation de composés chimiques, en particulier de composés fongicides, notamment de dérivés iodés de chromones, de préférence des composés de formule (I), ainsi que des composés intermédiaires utiles pour ces procédés.

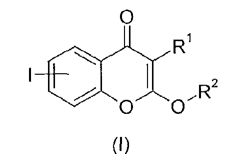

## Description

Cette invention concerne de nouveaux procédés de préparation de composés chimiques, en particulier de composés fongicides, notamment de dérivés iodés de chromones, de préférence des composés de formule (I), ainsi que des composés intermédiaires utiles pour ces procédés.

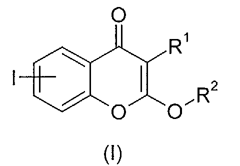

La présente invention a également pour objet un procédé de préparation de vinyl-alkoxydes à partir de vinyl-amines ; elle concerne plus particulièrement la préparation de 2-halogéno- et 2-alkoxychromones par réaction d'une 2-aminochromone avec un halogénure ou un alkoxyde de cuivre.
La présente invention a également pour objet un procédé de préparation de 2-aminochromones par réaction d'un salicylate d'alkyl avec un alkylnitrile et une base, suivie d'une cyclisation en milieu acide aqueux.

La demande de brevet européen EP-861 242, qui concerne des composés fongicides dérivés de coumarine ou de chromone, rappelle de manière générique des procédés de préparation de certains de ces composés. Toutefois, même si deux exemples de composés iodés sont divulgués, aucun procédé de synthèse spécifique de tels dérivés iodés n'est mentionné, pas plus que n'est suggéré un tel procédé amélioré.

On connaît dans l'art antérieur divers procédés permettant la préparation d'halogénures aromatiques à partir d'amines aromatiques primaires via une réaction de Sandmeyer. On connaît en particulier la préparation de sels de diazonium aromatiques (March's Advanced Organic Chemistry, 5^{ème} édition, John Wiley & sons, Inc., (2001), p816-817 et 936) ainsi que les réactions chimiques associées à de tels sels de diazonium (Chem. Rev., (1988), 88, 765-792). Cependant, aucun des procédés connus ne décrit de telles transformations en série éther vinylique selon le schéma ci-dessous dans lequel * représente un site libre, G1 représente un atome d'halogène ou un alkoxyde et pour lequel les structures sont linéaires ou cycliques qui peuvent être aromatiques ou non:

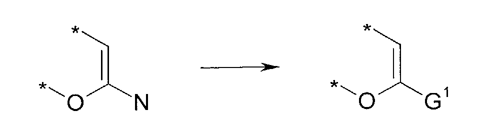

Au cours de la préparation des composés fongicides visés par la présente invention, on rencontre souvent les problèmes suivants:
- difficulté d'améliorer les procédés tant d'un point de vue technique que d'un point de vue économique;
- rendement faible;
- pureté insuffisante;
- difficulté du recyclage des réactifs employés;
- taux de conversion faible;
- réduction du nombre d'étapes;
- utilisations de réactifs plus économiques;
- amélioration de la simplicité et de la sûreté des réactions mises en oeuvre;
- limitation du nombre des sous-produits formés;
- limitations des réactions parasites.

La présente invention permet de solutionner en tout ou partie ces problèmes ou inconvénients.

Un premier aspect de cette invention concerne la préparation d'un composé de formule (I) 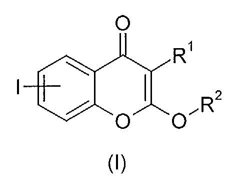 selon les étapes suivantes:
étape A:
   réaction d'un composé de formule (V) 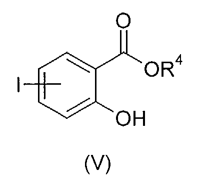 avec un nitrile de formule R¹CH₂CN et une base pour former le composé de formule (IV); 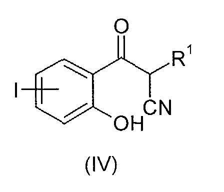
étape B:
   composé de formule (IV) qui est ensuite cyclisé en milieu acide aqueux pour former le composé de formule (III); 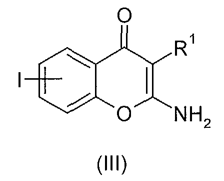
étape C:
   composé de formule (III) qui donne le composé de formule (II) via une réaction de diazotation au moyen d'un composé choisi parmi le nitrite de sodium en mélange avec un acide, les alkylnitrites, les alkylthionitrites et les alkylthionitrates, et décomposition au moyen d'un composé AZₙ; 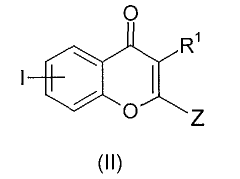
étape D:
   composé de formule (II) qui donne le composé de formule (I) par action d'un alcool en mélange avec une base;
dans les formules (I) à (V)
- R¹, R², R³ et R⁴, identiques ou différents, représentent un alkyl en C₁-C₁₀, alkenyl en C₁-C₁₀ ou alkynyl en C₁-C₁₀, un ou plusieurs carbo- ou hétérocycles ayant de 5 à 7 atomes, ces groupements pouvant être substitués ou non substitués;
- A représente un métal ou un sel métallique;
- Z représente un groupe choisi parmi CI, Br ou -OR³;
- n est égal à 0, 1 ou 2.

Lorsque les groupements ou radicaux des composés de formule (I) à (V) sont substitués, ils le sont de manière préférée par un ou plusieurs groupements qui peuvent être choisis indépendamment les uns des autres parmi les radicaux alkyle, alkényle, alkynyle, un atome d'halogène, le radical cyano, trialkylsilyle, alkoxy, alkylthio, hydroxy, nitro, amino, acyle, acyloxy, phényle, hétérocyclyle, phénylthio, phénoxy, hétérocyclyloxy, hétérocyclylthio et des dérivés oxydés et éventuellement substitués d'entités chimiques contenant un groupement thio.
Le terme hétérocyclyle comprend des groupements hétéroaryles ainsi que des groupements hétérocyclyles non-aromatiques qui peuvent être saturés ou insaturés.
Les groupements hétéroaryles sont généralement des noyaux à 5 ou 6 chaînons contenant jusqu'à 4 hétéroatomes choisis parmi azote, oxygène et soufre, éventuellement fusionnés à un noyau benzénique. Comme exemples de groupements hétéroaryles, on peut citer les groupements dérivés du thiophène, du furane, du pyrrole, du thiazole, de l'oxazole, de l'imidazole, de l'isothiazole, de l'isoxazole, du pyrazole, du 1,3,4-oxadiazole, du 1,3,4-thiadiazole, du 1,2,4-oxadiazole, du 1,2,4-thiadiazole, du 1,2,4-triazole, du 1,2,3-triazole, du tétrazole, du benzo[b]thiophène, du benzo[b]furane, de l'indole, du benzo[c]thiophène, du benzo[c]furane, de l'iso-indole, du benzoxazole, du benzothiazole, du benzimidazole, du benzisoxazole, du benzisothiazole, de l'indazole, du benzothiadiazole, du benzotriazole, du dibenzofurane, du dibenzothiophène, du carbazole, de la pyridine, de la pyrazine, de la pyrimidine, de la pyridazine, de la 1,3,5-triazine, de la 1,2,4-triazine, de la 1,2,4,5-tétrazine, de la quinoléine, de l'isoquinoléine, de la quinoxaline, de la quinazoline, de la cinnoline, de la 1,8-naphtyridine, de la 1,5-naphtyridine, de la 1,6-naphtyridine, de la 1,7-naphtyridine, de la phtalazine, de la pyridopyrimidine, de la purine ou de la ptéridine.
Les groupements hétérocyclyles non-aromatiques sont généralement des noyaux à 3, 5, 6 ou 7 chaînons contenant jusqu'à 3 hétéroatomes choisis parmi azote, oxygène et soufre, par exemple oxiranyle, thi-iranyle, thiazolinyle, dioxolanyle, 1,3-benzoxazinyle, 1,3-benzothiazinyle, morpholino, pyrazolinyle, sulfolanyle, dihydroquinazolinyle, pipéridinyle, phtalimido, tétrahydrofuranyle, tétrahydropyranyle, pyrrolidinyle, indolinyle, 2-oxopyrrolidino, 2-oxobenzoxazolin-3-yle ou tétrahydroazépinyle.
Les substituants, lorsqu'ils sont présents, sur les groupements phényles ou hétérocyclyles peuvent par exemple être des atomes d'halogène, CN, NO₂, SF₅, B(OH)₂, trialkylsilyle, acyle, O-acyle ou un radical E, OE ou S(O)ₙ E comme défini précédemment pour R¹ ou bien un radical amino éventuellement substitué ; ou bien deux groupements adjacents sur le cycle, ensemble avec les atomes auxquels ils sont attachés, forment un noyau carbocyclique ou hétérocyclique, qui peut être éventuellement substitué de manière similaire.

Le terme acyle comprend les résidus d'acides contenant du soufre ou du phosphore ainsi que les résidus d'acides carboxyliques. Des exemples de groupements acyles sont ainsi -COR⁵, -COOR⁵, -CONR⁵R⁶, -CON(R⁵)OR⁶, -COONR⁵R⁶, -CON(R⁵)NR⁶R⁷, -COSR⁵, -CSSR⁵, -S(O)qR⁵, - S(O)₂OR⁵, -S(O)_{q}NR⁵R⁶, -P(=L)(OR⁵)(OR⁶) ou -COOR⁵, dans lesquels R⁵, R⁶ et R⁷, qui peuvent être identiques ou différents, représentent l'atome d'hydrogène, un radical alkyle éventuellement substitué, cycloalkyle éventuellement substitué, cycloalkényle éventuellement substitué, alkényle éventuellement substitué, alkynyle éventuellement substitué, un groupement phényle éventuellement substitué ou hétérocyclyle éventuellement substitué, ou bien R⁵ et R⁶, ou R⁶ et R⁷, ensemble avec le(s) atome(s) au(x)quel(s) ils sont attachés, peuvent former un cycle, q représente 1 ou 2 et L représente O ou S.
Le cas échéant, les radicaux amino peuvent être substitués par exemple par un ou deux radicaux alkyles éventuellement substitués ou acyles éventuellement substitués, ou bien deux substituants peuvent former un cycle, de préférence un cycle de 5 à 7 chaînons, qui peut être substitué et qui peut contenir d'autres hétéroatomes, ainsi par exemple la morpholine.
Le cas échéant, l'homme du métier saura adapter les conditions de réaction selon les groupements présents, notamment en les protégeant convenablement.

La combinaison des étapes A et B, de même que les étapes C et D prises isolément constituent des procédés qui font également partie de la présente invention en tant que tels. Les réactifs, les conditions réactionnelles et les éventuelles variantes d'étapes sont analogues à ceux ou celles décrites pour le procédé de l'invention comportant les étapes A à D.

On peut préparer les composés de formule (V) de manière analogue à ce qui a été rapporté dans la littérature pour des 3-halogénosalicylates d'alkyle à partir des salicylates d'alkyle équivalents, pour exemple voir Pharm. J., (1947), 159, 182.

Lors de la réaction de condensation de l'étape A utilisant le composé de formule (V) pour donner le composé de formule (IV), on utilise avantageusement une base minérale ou organique, homogène ou hétérogène, de préférence un alkylamidure de lithium, par exemple le diisopropylamidure de lithium.
De manière avantageuse, la base utile à cette étape peut être constituée d'un mélange de telles bases. L'homme du métier saura déterminer le nombre et les quantités relatives de ces bases utilisées en mélange.
Généralement, le composé de formule (IV) est caractérisé mais n'est pas isolé.

Selon une variante A' de cette étape A, le nitrile de formule R¹CH₂CN peut être remplacé par un nitrile de formule R¹CXHCN, dans laquelle X représente un atome d'halogène et la base est alors remplacée par un métal, notamment le magnésium ou le zinc, voir notamment Synth. Commun. (1989), 19(9-10), 1649-53.

Au cours de l'étape B, le composé de formule (IV) donne le composé de formule (III). On emploie un acide inorganique ou organique; le chlorure d'ammonium, l'acide chlorhydrique ou l'acide acétique conviennent bien. On préfère l'acide acétique aqueux.

Lors de l'étape C, le composé de formule (III) donne le composé de formule (II) via une réaction de diazotation, pour exemple voir March's Advanced Organic Chemistry, 5^{ème} édition, John Wiley & sons, Inc., (2001), p816, suivie d'une réaction de décomposition.
Comme réactifs préférés utiles pour cette diazotation, on peut citer le nitrite de sodium, et les alkylnitrites, notamment les nitrites de t-butyl ou de methyl.
Lors de l'utilisation de NaNO₂, les acides préférés sont HCl, HBr, H₂SO₄.

De manière préférée, l'intermédiaire diazo peut être décomposé au moyen d'un composé AZₙ choisi parmi un halogénure ou un alkoxyde métallique, de préférence CuCl, CuBr, CuCl₂, CuBr₂, CuOR³ ou Cu(OR³)₂; CuCl₂ et CuBr₂ peuvent également être utilisés sous leur forme hydratée.
Les combinaisons suivantes sont particulièrement avantageuses:
- nitrites de t-butyl ou de methyl et Cu(OR³)₂ ou CuCl₂;
- nitrite de sodium avec HCl ou H₂SO₄ et CuOR³ ou CuCl.
Au cours de cette étape C, A représente donc de préférence le cuivre. D'autres métaux utiles pour cette étape sont mentionnés dans Chem. Rev., (1988), 88, 765-792.
Cette étape C peut également être mise en oeuvre en utilisant NaNO₂ avec CISiMe₃ afin d'aboutir à Z représentant CI; l'homme du métier saura adapter les conditions d'une telle réaction de Sandmeyer connue en tant que telle (Tet. Letters, 33 (22), 3167-3168 (1992)).
De manière similaire, NaNO₂ en mélange avec du cuivre métallique et KI peuvent être mis en oeuvre lors de cette étape C, dans ce cas Z représente I.
De même, HBF₄ en mélange avec NaNO₂ peut être employé. Z représente alors F.

Lors de l'étape D, le composé de formule (II) donne le composé de formule (I). On emploie un alcool de formule R²OH, dans laquelle R² représente un groupement tel que ceux définis plus haut, de préférence un alkyl en C₁-C₁₀, encore plus préférentiellement le groupement n-butyl. Le choix de la base employée en vue de former l'alcoolate est à la portée de l'homme du métier qui pourra notamment se tourner vers un hydroxyde ou un hydrure alcalin ou acalino terreux, un métal alcalin ou alcalino terreux, de préférence KOH, NaH ou le sodium métallique.

Un autre aspect de la présente invention concerne un procédé encore amélioré de préparation de ce même composé de formule (I) obtenu directement par diazotation et décomposition d'un composé de formule (III) au moyen d'un composé AZₙ qui est, de préférence, un alkoxyde métallique.
Dans ce cas, les étapes C et D du procédé de l'invention sont remplacées par une unique étape C'.
Les différents substituants des composés ont alors les mêmes significations que précédemment.

Une autre variante du procédé de l'invention consiste à remplacer l'étape D par une étape D'. Dans ce cas on utilise un composé de formule (II) dans lequel Z représente un groupement - OR³, il est alors possible de déplacer ce groupement par un groupement analogue plus approprié. Par exemple, dans le cas où Z est le groupement ethoxy, on pourra le déplacer par un groupement butoxy qui peut être introduit par traitement avec du butylate de sodium. La mise en oeuvre d'une telle substitution constitue un procédé supplémentaire de la présente invention pour la préparation du composé de formule (I) à partir d'un composé de formule (II).

Les procédés de l'invention qui sont particulièrement préférés permettent la préparation de composés de formule (I) au moyen des composés de formules (II) à (V) dans lesquelles les caractéristiques suivantes sont présentes seules ou en combinaison:
- l'atome d'iode est en position 6 de la chromone;
- R¹ représente un alkyl en C₁-C₁₀, de préférence un n-propyl;
- R² représente un alkyl en C₁-C₁₀, de préférence un n-butyl;
- R⁴ représente un alkyl en C₁-C₁₀, de préférence un methyl;
- A représente Cu;
- Z représente un atome d'halogène, de préférence CI ou Br, ou le groupement -OR³ dans lequel R³ représente un groupement methyl ou n-butyl.
De manière toute préférée, on met en oeuvre les procédés de l'invention pour la préparation d'un composé de formule (I) dans laquelle R¹ représente un n-propyl et R² représente un n-butyl.

Comme composés avantageusement préparés au moyen des procédés de l'invention, on peut notamment citer les composés de formule (1) dont le nom chimique est le suivant:
- 2-butoxy-6-iodo-3-propyl-4H-1-benzopyran-4-one,
- 2-ethoxy-6-iodo-3-propyl-4H-1 -benzopyran-4-one,
- 6-iodo-2-propoxy-3-propyl-4H-1-benzopyran-4-one,
- 2-but-2-ynyloxy-6-iodo-3-propyl-4H-1-benzopyran-4-one,
- 6-iodo-2-(1-methyl-butoxy)-3-propyl-4H-1-benzopyran-4-one,
- 2-but-3-enyloxy-6-iodo-3-propyl-4H-1-benzopyran-4-one,
- 3-butyl-6-iodo-2-isopropoxy-4H-1-benzopyran-4-one,
- 6-iodo-3-propyl-2-(tetrahydro-pyran-4-yloxy) -4H-1-benzopyran-4-one,
- 6-iodo-3-propyl-2-(2,2,2-trifluro-ethoxy) -4H-1-benzopyran-4-one.

Par ailleurs l'homme du métier saura adapter les procédés de l'invention à la préparation des composés de formule (I) à (V) sous forme de leurs éventuels isomères géométriques et/ou optiques, purs ou en mélanges, en toutes proportions, y compris le ou les éventuels mélanges racémiques, leurs éventuels N-oxydes, sels d'addition à un acide, acceptables pour une utilisation dans le domaine de la protection des cultures, et leurs éventuels complexes métalliques et/ou métalloïdiques, acceptables pour une utilisation dans le domaine de la protection des cultures.

Les procédés de l'invention viennent d'être décrits en relation avec des dérivés de chromone iodés. Toutefois, ces procédés pourront être adaptés pour la préparation d'autres dérivés halogénés de chromone.

Un aspect supplémentaire de la présente invention concerne des composés intermédiaires utiles pour les procédés de préparation selon l'invention.
Ces composés sont les composés de formule (III) ainsi que les composés de formule (II) dans lesquelles les différents substituants ont les mêmes significations que précédemment.

Parmi les composés intermédiaires de formule (III), on préfère ceux dans lesquels les caractéristiques suivantes sont présentes seules ou en combinaison:
- l'atome d'iode est en position 6 de la chromone;
- R¹ représente un groupement alkyl en C₁-C₁₀; de préférence un groupement n-propyl.

Parmi les composés intermédiaires de formule (II), on préfère ceux dans lesquels les caractéristiques suivantes sont présentes seules ou en combinaison:
- l'atome d'iode est en position 6 de la chromone;
- R¹ représente un groupement alkyl en C₁-C₁₀;
- Z représente un halogène ou un groupement -OR³.
Plus préférentiellement, R¹ représente un groupement n-propyl et Z représente le chlore ou le brome.

Les exemples suivants sont donnés à titre non limitatif en vue d'illustrer d'une façon supplémentaire la mise en oeuvre de l'invention.

### Exemple 1 : préparation du 2-amino-6-iodo-3-propylchromone

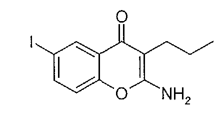

Une solution de 8,4 ml (0.06 mole) de diisopropylamine dans 20ml de THF anhydre, refroidie à - 20°C est traitée par l'addition de 30ml (0.06 mole) de *n*-butyllithium 2M en solution dans hexanes en 15 minutes. La solution est maintenue sous agitation à cette température pendant 5 minutes et 2,10 ml (0.02 mole) de valéronitrile sont additionnés à -20°C en 5 minutes. La solution est maintenue sous agitation à -20°C pendant 15 minutes et une solution de 5,84g (0.02 mole) de 4-iodosalicylate d'éthyle dans 20ml de THF anhydre est additionnée en 15 minutes. La suspension est réchauffée à 5°C et maintenue sous agitation pendant 45 minutes puis hydrolysée avec 15 ml d'une solution aqueuse saturée de NH₄Cl. La solution biphasique est maintenue sous agitation pendant 2 heures. La phase aqueuse est alors éliminée, la phase organique est lavée avec 2x20 ml d'eau puis 20 ml d'une solution aqueuse saturée de NaCl. La phase organique est séchée sur MgSO₄, filtrée et concentrée à sec, ce qui donne 7,25g d'un solide jaune (70%). Le produit cristallisé dans l'acétonitrile a un point de fusion de 186-188°C.

### Exemple 2 : préparation du 2-chloro-6-iodo-3-propylchromone

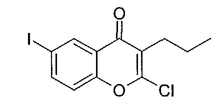

A une suspension de 1,98g (6 mmoles) de 2-amino-6-iodo-3-propylchromone, 1,22g (7,2 mmoles) de CuCl2, 2H2O dans 60 ml d'acétonitrile, refroidie à 0°C est additionné 0.86 ml (7,2 mmoles) de *tert*-butylnitrite en 10 minutes. Le milieu réactionnel est réchauffé à 20°C et maintenu sous agitation à cette température pendant 3 heures. La solution est hydrolysée par l'addition de 60 ml d'eau et extraite avec 2x50 ml d'acétate d'éthyle. Les phases organiques sont réunies et lavées avec 20 ml de NH₄OH, 2x50 ml d'eau puis 50 ml d'une solution aqueuse saturée de NaCl. La phase organique est séchée sur MgSO₄, filtrée et concentrée à sec. Ce qui donne 2,04g d'un solide marron (78% de rendement théorique), de point de fusion 103-105°C.

### Exemple 3 : préparation du 2-butoxy-6-iodo-3 propylchromone

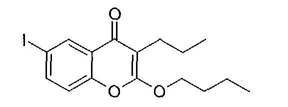

A une solution de 1,05g (3 mmoles) de 2-chloro-6-iodo-3-propylchromone dans 10 ml de dichloromethane sont additionnés, à 20°C, 2,4 ml (4,3 mmoles) d'une solution à 20% de n-butylate de sodium dans le *n*-butanol. La solution est maintenue à 20°C pendant 2 heures puis chauffée à 40°C pendant 4 heures. Le milieu réactionnel est hydrolysé avec 10ml d'eau et la phase organique est lavée avec 2×10ml d'eau, séchée sur MgSO₄ filtrée et concentrée à sec. Ce qui donne 0,95g d'une huile brune qui cristallise. Le produit cristallisé dans l'acétonitrile a un point de fusion de 69-71°C.

### Exemple 4 : préparation du 6-iodo-2-méthoxy -3-propylchromone

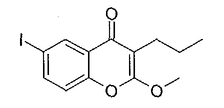

A une suspension de 0,31g (0,95 mmole) de 2-amino-6-iodo-3-propylchromone, 0,15g (1.2 mmole) de Cu(OMe)₂ dans 2 ml d'acétonitrile, est additionné, à 20°C, 0,14 ml (1,2 mmole) de *tert*-butylnitrite en 5 minutes. Le milieu réactionnel est chauffé à 60°C et maintenu sous agitation à cette température pendant 3 heures. Après retour à température ambiante, le mélange réactionnel est hydrolysé par l'addition de 1 ml d'eau et extrait avec 2x5 ml d'acétate d'éthyle. Les phases organiques sont réunies et lavées avec 5 ml de NH₄OH 2x5 ml d'eau puis 5 ml d'une solution aqueuse saturée de NaCl. La phase organique est séchée sur MgSO₄ filtrée et concentrée à sec.

### Exemple 5 : préparation du 2-n-butoxy-6-iodo-3-propylchromone

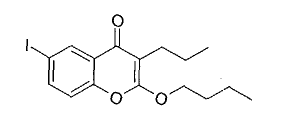

A une solution de 0,27g (0,76 mmole) de 2-éthoxy-6-iodo-3-propylchromone dans 2.5 ml de n-butanol, est additionné, à 20°C, 0,5 ml (0.91 mmole) d'une solution à 20% (w/w) de n-butylate de sodium dans le n-butanol en 5 minutes. Le milieu réactionnel est maintenu sous agitation à 20°C pendant 2 heures. Le mélange réactionnel est traité par l'addition de 2 ml d'une solution aqueuse de HCl 0.1N et extrait avec 10 ml de dichloromethane. La phase organique est extraite et lavée avec 2x5 ml d'eau. La phase organique est séchée sur MgSO₄ filtrée et concentrée à sec. Ce qui donne 0,24g d'une huile brune qui cristallise.

## Revendications

1. Procédé de préparation d'un composé de formule (I) 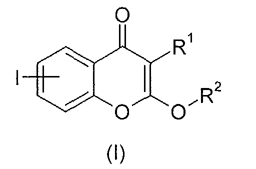 selon les étapes suivantes:
étape A:
réaction d'un composé de formule (V) 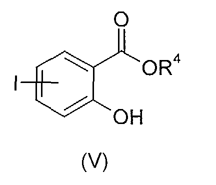 avec un nitrile de formule R¹CH₂CN et une base pour former le composé de formule (IV); 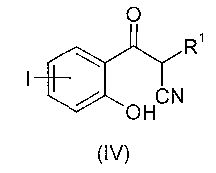
étape B:
composé de formule (IV) qui est ensuite cyclisé en milieu acide aqueux pour former le composé de formule (III); 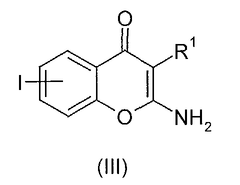
étape C:
composé de formule (III) qui donne le composé de formule (II) via une réaction de diazotation au moyen d'un composé choisi parmi le nitrite de sodium en mélange avec un acide, les alkylnitrites, les alkylthionitrites et les alkylthionitrates; et décomposition au moyen d'un composé AZₙ; 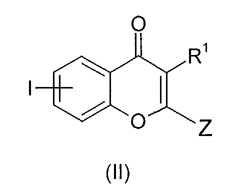
étape D:
composé de formule (II) qui donne le composé de formule (I) par action d'un alcool en mélange avec une base;
dans les formules (I) à (V)
• R¹, R², R³ et R⁴, identiques ou différents, représentent un alkyl en C₁-C₁₀, alkenyl en C₁-C₁₀ ou alkynyl en C₁-C₁₀, un ou plusieurs carbo- ou hétérocycles ayant de 5 à 7 atomes, ces groupements pouvant être substitués ou non substitués;
• A représente un métal ou un sel métallique;
• Z représente un groupe choisi parmi Cl, Br ou -OR³;
• n est égal à 0, 1 ou 2.

2. Procédé selon la revendication 1 pour lequel la base employée lors de l'étape A est le diisopropylamidure de lithium.

3. Procédé selon l'une ou l'autre des revendications 1 et 2 pour lequel l'acide employé lors de l'étape B est l'acide acétique.

4. Procédé selon l'une ou l'autre des revendications 1 à 3 pour lequel A est le cuivre.

5. Procédé selon l'une ou l'autre des revendications 1 à 4 dont l'étape C met en oeuvre du nitrite de t-butyl ou de methyl.

6. Procédé selon l'une ou l'autre des revendications 1 à 4 pour lequel AZₙ représente Cu(OR³)₂ ou CuCl₂.

7. Procédé selon l'une ou l'autre des revendications 1 à 4 dont l'étape C met en oeuvre du nitrite de t-butyl ou de methyl et pour lequel AZₙ représente Cu(OR³)₂ ou CuCl₂.

8. Procédé selon l'une ou l'autre des revendications 1 à 4 dont l'étape C met en oeuvre du nitrite de sodium en mélange avec HCl ou H₂SO₄.

9. Procédé selon l'une ou l'autre des revendications 1 à 4 pour lequel AZₙ représente Cu(OR³) ou CuCl.

10. Procédé selon l'une ou l'autre des revendications 1 à 4 dont l'étape C met en oeuvre du nitrite de sodium en mélange avec HCl ou H₂SO₄ et pour lequel AZₙ représente Cu(OR³) ou CuCl.

11. Procédé selon l'une ou l'autre des revendications 1 à 10 dont l'étape D met en oeuvre un alcool de formule R²OH dans laquelle R² est un alkyl en C₁-C₁₀.

12. Procédé selon la revendication 11 pour lequel R² représente le n-butyl.

13. Procédé selon l'une ou l'autre des revendications 1 à 11 dont l'étape D est remplacée par une étape D' et dans lequel on utilise un composé de formule (II) dans lequel Z représente un groupement -OR³, qui est déplacé par un groupement analogue plus approprié.

14. Procédé selon la revendication 13 pour lequel Z est le groupement ethoxy qui est déplacé par un groupement butoxy introduit par traitement avec du butylate de sodium.

15. Procédé selon l'une ou l'autre des revendications 1 à 14 dont l'étape A est remplacée par une étape A' et dans lequel est mis en oeuvre un nitrile de formule R¹CXHCN, dans laquelle X représente un atome d'halogène, avec comme base, un métal choisi parmi le magnésium ou le zinc.

16. Procédé selon l'une ou l'autre des revendications 1 à 15 pour lequel les caractéristiques suivantes sont présentes seules ou en combinaison:
• l'atome d'iode est en position 6 de la chromone;
• R¹ représente un alkyl en C₁-C₁₀, de préférence un n-propyl;
• R² représente un alkyl en C₁-C₁₀, de préférence un n-butyl;
• R⁴ représente un alkyl en C₁-C₁₀, de préférence un methyl;
• A représente Cu;
• Z représente Cl ou Br, ou le groupement -OR³ dans lequel R³ représente un groupement methyl ou n-butyl.

17. Procédé selon l'une ou l'autre des revendications 1 à 16 pour la préparation d'un composé de formule (I) dans laquelle R¹ représente un n-propyl et R² représente un n-butyl.

18. Procédé de préparation d'un composé de formule (III) 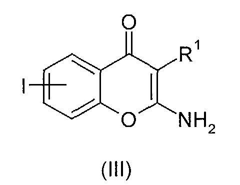 selon les étapes suivantes:
réaction d'un composé de formule (V) 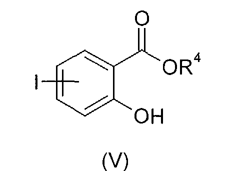 avec un nitrile de formule R¹CH₂CN et une base pour former le composé de formule (IV); 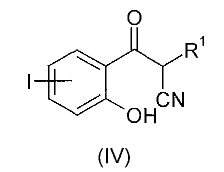 composé de formule (IV) qui est ensuite cyclisé en milieu acide aqueux pour former le composé de formule (III);
dans les formules (III) à (V)
• R¹, R², R³ et R⁴, identiques ou différents, représentent un alkyl en C₁-C₁₀, alkenyl en C₁-C₁₀ ou alkynyl en C₁-C₁₀, un ou plusieurs carbo- ou hétérocycles ayant de 5 à 7 atomes, ces groupements pouvant être substitués ou non substitués.

19. Procédé de préparation d'un composé de formule (II) 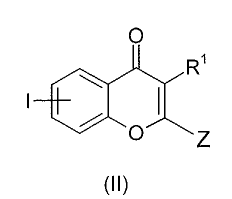 à partir d'un composé de formule (III) qui donne le composé de formule (II) via une réaction de diazotation 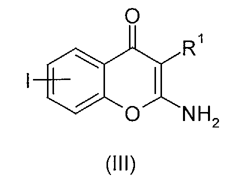 au moyen d'un composé choisi parmi le nitrite de sodium en mélange avec un acide, les alkylnitrites, les alkylthionitrites et les alkylthionitrates; et décomposition au moyen d'un composé AZₙ;
dans les formules (II) et (III)
• R¹ et R³, identiques ou différents, représentent un alkyl en C₁-C₁₀, alkenyl en C₁-C₁₀ ou alkynyl en C₁-C₁₀, un ou plusieurs carbo- ou hétérocycles ayant de 5 à 7 atomes, ces groupements pouvant être substitués ou non substitués;
• A représente un métal ou un sel métallique;
• Z représente un groupe choisi parmi Cl, Br ou -OR³;
• n est égal à 0, 1 ou 2.

20. Procédé de préparation d'un composé de formule (I) 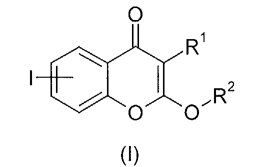 à partir d'un composé de formule (II) qui donne le composé de formule (I) 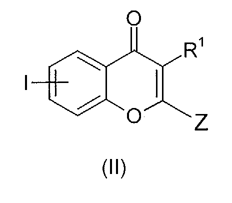 par action d'un alcool en mélange avec une base;
dans les formules (I) et (II)
• R¹ et R², identiques ou différents, représentent un alkyl en C₁-C₁₀, alkenyl en C₁-C₁₀ ou alkynyl en C₁-C₁₀, un ou plusieurs carbo- ou hétérocycles ayant de 5 à 7 atomes, ces groupements pouvant être substitués ou non substitués;
• Z représente un groupe choisi parmi CI, Br ou -OR³.

21. Composé de formule (III) 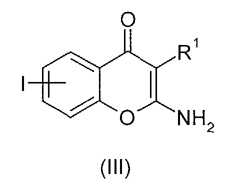 dans laquelle R¹ représente un alkyl en C₁-C₁₀, alkenyl en C₁-C₁₀ ou alkynyl en C₁-C₁₀, un ou plusieurs carbo- ou hétérocycles ayant de 5 à 7 atomes, ces groupements pouvant être substitués ou non substitués.

22. Composé selon la revendication 21 de formule (III) dans laquelle les caractéristiques suivantes sont présentes seules ou en combinaison:
• l'atome d'iode est en position 6 de la chromone;
• R¹ représente un groupement alkyl en C₁-C₁₀.

23. Composé selon l'une ou l'autre des revendications 21 et 22 de formule (III) dans laquelle R¹ représente le groupement n-propyl.

24. Composé de formule (II) 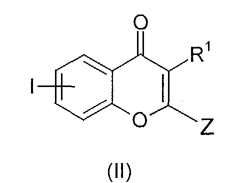 dans laquelle
• R¹ et R³, identiques ou différents, représentent un alkyl en C₁-C₁₀, alkenyl en C₁-C₁₀ ou alkynyl en C₁-C₁₀, un ou plusieurs carbo- ou hétérocycles ayant de 5 à 7 atomes, ces groupements pouvant être substitués ou non substitués;
• Z représente un groupe choisi parmi CI, Br ou -OR³.

25. Composé selon la revendication 24 de formule (II) dans laquelle les caractéristiques suivantes sont présentes seules ou en combinaison:
• l'atome d'iode est en position 6 de la chromone;
• R¹ représente un groupement alkyl en C₁-C₁₀;
• Z représente un halogène ou un groupement -OR³.

26. Composé selon l'une ou l'autre des revendications 24 et 25 de formule (II) dans laquelle R¹ représente un groupement n-propyl et Z représente le chlore ou le brome.
